# EUROPEAN PATENT SPECIFICATION

(11) **EP 1 372 621 B1**
(45) Date of publication and mention of the grant of the patent: **11.06.2008**
(21) Application number: 02720710.9
(22) Date of filing: 05.04.2002
(51) Int. Cl.: A61K 31/00, A61K 31/496, A61K 31/192, A61K 31/216, A61K 48/00, A61K 39/395, A61P 3/10

(54) **NOVEL USE OF TYROSINE KINASE FRK INHIBITOR**
NEUE VERWENDUNG VON FRK-TYROSINEKINASE INHIBITOREN
NOUVELLE UTILISATION D'UN INHIBITEUR DE KINASE DE TYROSINE FRK

(30) Priority: 06.04.2001 SE 0101230
(43) Date of publication of application: 02.01.2004
(73) Proprietor: Innoventus Project AB, 751 83 Uppsala (SE)
(72) Inventor: WELSH, Michael, S-752 41 Uppsala (SE); ANNEREN, Cecilia, S-752 43 Uppsala (SE)
(74) Representative: Dahnér, Christer
(86) International application number: PCT/SE2002/000682
(87) International publication number: WO 2002/080894

(56) References cited:
- WO-A1-95/21613
- WO-A1-99/21859
- US-A- 5 834 504
- ANNEREN C. ET AL.: 'Transgenic mice expressing the tyrosine kinase gtk exhibit increased cytokine-induced beta-cell toxicity' DIABETOLOGIA vol. 43, no. SUPPL. 1, 2000, page 475, A123
- WELSH MICHAEL ET AL.: 'Role of tyrosine kinase signaling for B-cell replication and survival' UPPSALA J. MED. SCI. vol. 105, 2000, pages 7 - 15

## Description

The present invention relates to a novel use of a tyrosine kinase inhibitor or blocking agent. More precisely, it relates to use of an inhibitor of a FRK tyrosine kinase for production of a drug for prevention /treatment of type I diabetes mellitus.

Type 1 diabetes mellitus results from the autoimmune destruction of the pancreatic β-cell and this disease has up to date been treated with a regimen of life-long insulin injections. Numerous agents, such as viruses, autoreactive antibodies, macrophages, T-cells, cytokines, nitric oxide and free oxygen radicals have all been proposed to mediate β-cell toxicity. This process is thought to proceed over a long time period, maybe years, and diabetes finally ensues when the remaining β-cells cannot maintain glucose homeostasis. Nevertheless, recent results suggest that a significant proportion of the β-cell mass is retained when signs of diabetes become overt, and this fact is the basis for the concept of disease intervention to rescue the remaining β-cells. Different strategies have been proposed for such intervention therapy: One involves the attenuation of the destructive autoimmune response by cyclosporin treatment but was abandoned despite promising initial results due to the toxic side-effects of drug treatment. Another attempt to allow the β-cells to recover in type 1 diabetes occurs by inducing " β-cell rest", which translates into maintaining β-cells in a low functional state under conditions of high stress by simultaneous insulin and diazoxide administration. Although this did not cure diabetes, it preserved residual insulin secretion. Additional approaches for intervention therapy currently investigated are through the administration of nicotinamide or insulin to newly diagnosed patients. Besides drug therapy, gene therapy to construct β-cells that are more resistant to the autoimmune attack is being considered.

Immune-mediated local release of cytokines, particularly IL-1β, IFN-γ and TNF-α have all been reported to cause severe β-cell destruction and contribute to the development of type 1 diabetes (Mandrup-Poulsen, 1996). Although much work has focused on their mode of action, certain aspects of their β-cell destructive effects have remained elusive. It is generally believed that cytokine-induced β-cell destruction is at least partially caused by the induction of iNOS, inducible nitric oxide synthase, which generates NO and has deleterious effects on the β-cells. However, it may also be the case that certain cytotoxic events may occur independently of NO-generation (Liu et al, 2000). Thus, it can be stated that the precise mechanism by which cytokines cause β-cell destruction remains elusive.

In WO00100981 inhibitors against JAK 3 kinase are described as useful for treating leukemia and lymphoma. Also treatment of diabetes is mentioned with these inhibitors. However, JAK 3 kinases are present in many cell types and therefore inhibitors against these kinases are not selective for inhibition in β-cells.

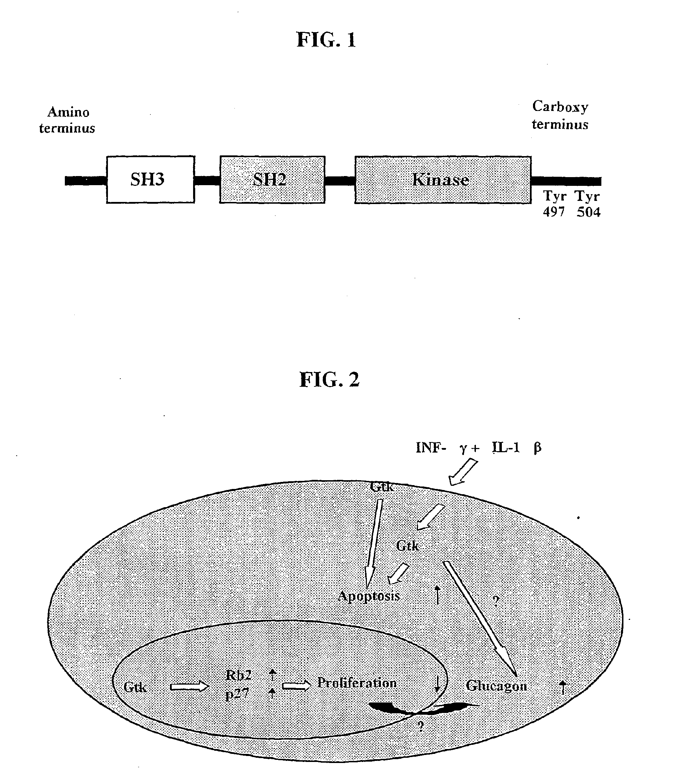

In 1995, a novel tyrosine kinase sequence in the RINm5F insulinoma cDNA was detected and cloned (Öberg & Welsh, 1995) and named BSK. BSK is identical to IYK and homologous to the rat sequence GTK and human sequence FRK/RAK. For practical reasons, BSK/IYK was renamed to mouse GTK. GTK has the characteristics of a Src-like tyrosine kinase with a partial myristoylation signal in its N-terminus, SH2 and SH3 (Src homology- 2,- 3) domains, a tyrosine kinase domain and regulatory tyrosines in its C-terminus. In mouse GTK, there are two potential regulatory tyrosines corresponding to tyrosine-527 in Src; tyrosine 497 and 504, with the latter being the more plausible candidate (Fig. 1). Mutation of these sites in order to constitutively activate mouse GTK was performed, and the mutants were stably expressed in NIH3T3 fibroblast cells (Öberg-Welsh et al, 1998). Whereas the Y497F- and Y504F-mutants each alone did not affect cell proliferation, the Y497/504F-double mutant decreased cell proliferation by increasing the number of cells in the G1 phase of the cell cycle. This indicates regulatory roles of both the 497 and 504 tyrosines. The Y504F mutation increased kinase activity in analogy with Y527 in Src, whereas the Y497F mutation caused a translocation of mouse GTK to the nucleus, and both these effects appear important for the inhibition of proliferation. When mouse GTK (wild-type and mutants) was overexpressed in the insulinoma RINm5F cells (Annerén & Welsh, 2000), inhibitory effects on cell replication were detected. Unlike in the NIH3T3 cells, the Y504F-mutant was also effective besides the Y497/504F-mutant in inhibiting cell proliferation by increasing the number of cells in the G1-phase of the cell cycle. The expressed Y504F-GTK mutant was partly detected in the nucleus, in contrast to what was observed in the NIH3T3 cells. The effects of mouse GTK expression were paralleled by increased expression of the cell cycle regulatory proteins p27^{Kip1} and p130 Rb2, thus-providing an explanation for the inhibitory effects on cell proliferation. The Y504F and Y497/504F-mutants conferred decreased viability in response to exposure to the cytokines IL-1β + IFN-γ. Finally, the expression of the islet hormones glucagon and insulin was assessed in the RINm5F cells expressing the mouse GTK mutants. All clones expressing the Y504F- and the Y497/504F-mutants showed elevated glucagon mRNA contents, whereas the Y497/504F-double mutant decreased the insulin mRNA level.
No in vivo studies have been made before the priority date of the present invention.

### Brief description of the drawings

**Fig. 1** is a schematic view of the GTK structure, indicating the positions of regulatory tyrosines located in the C-terminus.
**Fig. 2** is a schematic view showing possible roles of GTK for cell function.
**Fig. 3** is a bar chart showing the effects of different cytokines on cell viability in transgenic islets expressing GTK.
**Fig. 4** is a schematic view of a model for cytokine-induced β-cell death; and
**Fig. 5** is a schematic view of a hypothetical model for how GTK-inhibition could prevent cytokine-induced β-cell death in type I diabetes.

Fig. 2 depicts a possible role of GTK for cell function. GTK may be found both in the nucleus, at the plasma membrane and in the cytoplasm. Nuclear GTK increases the p27^{Kip1} and p130 Rb2 levels, and this decreases cell proliferation by inhibiting the cell cycle. GTK participates in the cytotoxicity of the cytokines IL-1β + IFN-γ, and this could reflect an action of GTK at the plasma membrane in proximity with the cytokine receptors or a later cytoplasmic event in the signaling pathways of these cytokines. The increase in glucagon gene expression is either the consequence of decreased cell proliferation or signaling from cytoplasmic GTK.

In order to further assess the role of mouse GTK for differentiation, neurite outgrowth in PC12 cells was assessed (Annerén et al, 2000). It was observed that PC12 cells stably overexpressing GTK exhibit a larger fraction of cells with neurites as compared to control cells, and this response is not accompanied by an increased ERK activity. GTK expression induces a NGF-independent Rap1 activation, probably through altered CrkII signaling. We observe increased CrkII complex formation with p130Cas, focal adhesion kinase (FAK) and SHB in PC12-GTK cells. The expression of GTK also correlates with a markedly increased content of FAK, phosphorylation of the adaptor protein SHB and an association between these two proteins. Inhibition of the Rap 1 pathway reduces the GTK-dependent neurite outgrowth. These data suggest that GTK participates in a signaling pathway involving SHB, FAK and Rap1, that induces differentiation in PC12 cells.

According to the present invention (see below), it has been found that GTK (BSK, IYK, FRK, RAK) tyrosine kinases mediate cytokine-induced β-cell destruction, and thus inhibition of these proteins or genes encoding them could attenuate the progression of type 1 diabetes. This inhibition may be directly or indirectly, for example by inhibition of the transcription or translation process. The inhibition of this tyrosine kinase comprises a novel approach for intervention therapy.

Thus, the invention relates to the use of, the inhibitor of tyrosine kinase SU4984 for the production of a drug for prevention and/or treatment of type I diabetes mellitus, wherein the inhibitor is against FRK tyrosine kinase in pancreatic β-cells. The inhibitor affects FRK tyrosine kinase activity directly or imdirectly.

The inhibitor may affect the cellular content of the FRK tyrosine kinase. The inhibitor may also, directly or indirectly, affect FRK tyrosine kinase signaling mechanisms present in pancreatic β-cells.

The invention herein is intended for human use. Thus, the preferred tyrosine kinase is the human FRK tyrosine kinase. Therefore, the applications of GTK below are also intended to encompass other equivalents of GTK, such as BSK, IYK, RAK, and especially FRK.

In a second aspect, the invention relates to use of FRK or the gene encoding the same, in an assay to screen for compounds for treatment of diabetes, which interfere with FRK activity in pancreatic β-cells.

In a third aspect, the invention relates to the use of FRK or the gene encoding the same for a biological or chemical assay for identifying compounds for treatment of diabetes in humans. The assay comprises FRK (or equvialents thereof) or genes encoding it, as molecular targets, in for example high throughput screening for identifying compounds interfering with FRK activity in pancreatic β-cells.

### EXPERIMENTAL PART

The above described effects of GTK have been described in cell lines, and to address the importance of GTK for β-cell function in vivo, a transgenic mouse expressing active GTK (Y504F) under the control of the insulin promoter to target expression to the β-cells has been generated. Control and GTK-transgenic CBA mice were used for determination of the *in vivo* glucose tolerance and the relative insulin-positive area. Isolated islets from both groups were cultured in the absence and presence of cytokines and viability and protein expression were assessed. The β-cell mass of GTK-transgenic mice was increased as a consequence of a larger pancreas and an increased relative β-cell area. Extracellular signal-regulated kinase (ERK) 1/2, p38 mitogen activated protein kinase (MAPK), c-Jun NH₂-terminal kinase (JNK) and Akt were all activated by cytokines but GTK-transgenic islets contained higher basal levels of phosphorylated ERK1/2 and lower basal levels of phosphorylated p38 compared with the control islets. The total amount of activated MAPKs was however higher in the cytokine-stimulated transgenic islets compared with the control islets due to increased levels of phospho-ERK1/2. Moreover, the proline-rich tyrosine kinase (PYK) 2 (also named RAFTK/CAK β/CADTK) levels were elevated in response to a 24-hour exposure to cytokines in control islets but not in the GTK-transgenic islets. (Annerén & Welsh 2001).

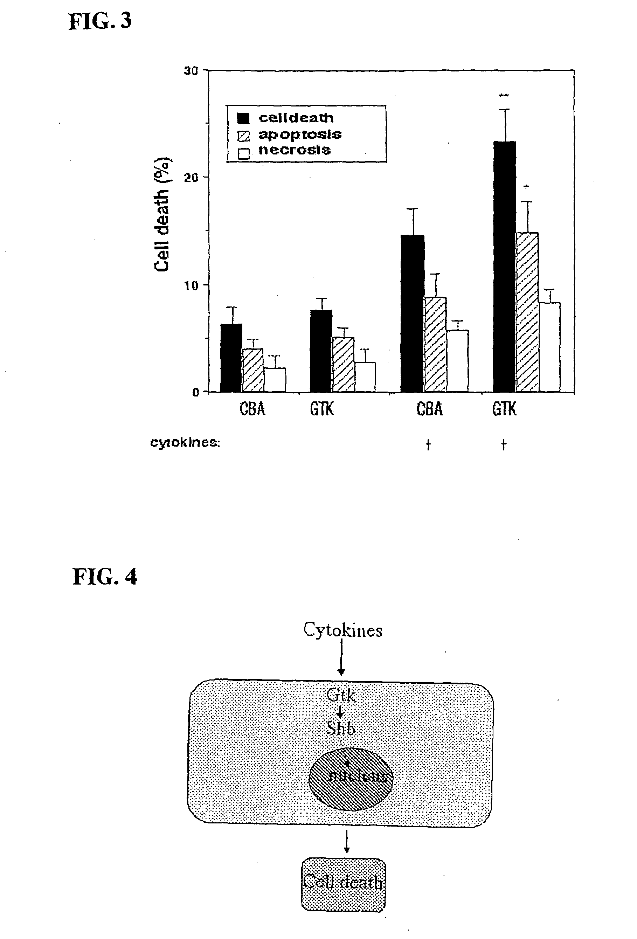

Role of GTK for cytokine-induced β-cell destruction: Islets isolated from the transgenic animals exhibited reduced viability in response to cytokines (Fig. 3) that could not be explained by higher levels of nitric oxide compared with control islets. This finding was in line with what was observed in the GTK-expressing RINm5F cells. Furthermore, a similar phenotype was observed in a SHB-transgenic mouse (Welsh et al, 1999), in which the SHB-overexpressing β-cells also displayed increased cell death upon exposure to cytokines. SHE has in several other systems been shown to induce apoptosis under certain conditions (Welsh et al, 2000). If SHB is downstream of GTK in β-cells as in the PC12 cells, the following model for cytokine-induced toxicity can be proposed: Cytokines, via unknown mechanisms, activate the GTK tyrosine kinase, which then phosphorylates SHB and thus causes an apoptotic response (see Fig. 4) possibly through activation of the different MAPK-pathways.

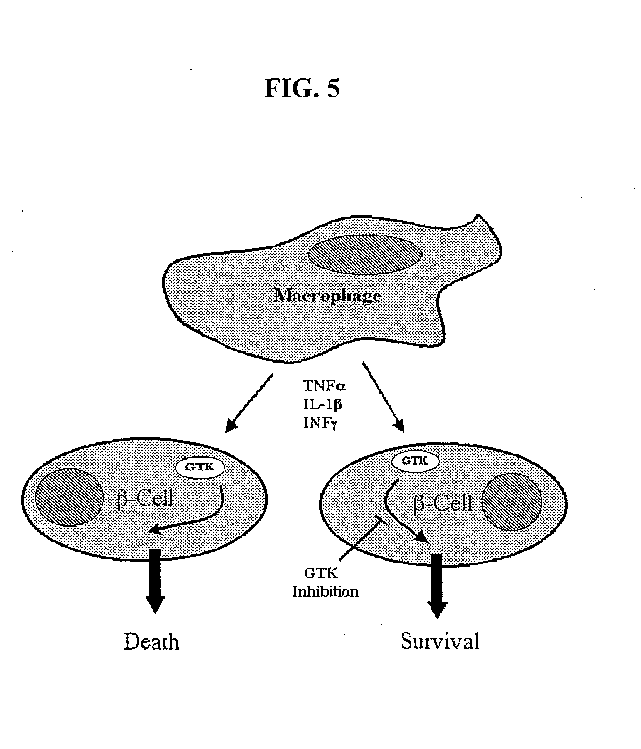

According to the present invention, GTK mediates cytokine-induced β-cell destruction, and by inhibiting this kinase during the initial phases of type 1 diabetes, further progression of the disease is attenuated. Many possibilities for specifically inhibiting GTK kinase activity can be envisaged. Thus a novel approach for intervention therapy is to administer a pharmacological agent that causes specific inhibition of the GTK kinase during the early phases of type 1 diabetes (see Fig. 5).

### BASIS FOR INHIBITION OF GTK (BSK, IYK, FRK, RAK) IN VIVO:

**1. Inhibition of GTK(BSK, TYK, FRK, RAK)-gene expression by gene therapy:** By gene transfer techniques, it is possible to obtain β-cell-specific inhibition of GTK kinase activity. Two possibilities exist: By expression of a dominant-negative GTK-product in β-cells or by expressing an anti-sense (RNAi) construct. In either case, the insulin-promoter is used to direct expression to β-cells. The β-cell-specific expression vector is well characterized and has been used successfully by us (Welsh et al. 1999). The RIP-vector contains the rat insulin 2 promoter, splice and polyA signals (Hanahan, 1984). The GTK cDNA is inserted into the unique BamHI site in the wrong orientation. The relevant portions of the construct (promoter, cDNA, splice and polyA sites) are then transferred into an appropriate adenoviral gene transfer vector. These usually employ the CMV promoter for expression, but it will be deleted and replaced by the RIP-GTK-antisense sequences. Alternatively, kinase-inactive GTK (K269A) will likewise be inserted into the RIP-vector and transferred to the adenoviral gene transfer vector. After construction of viral particles, islet cells are transfected in vitro. Expression of the GTK-antisense in β-cells will inhibit endogenous GTK translation, and thus no GTK protein will be formed. Expression of kinase-inactive GTK in β-cells will cause displacement of the endogenously expressed active GTK protein from its active intracellular sites and thus ablate the normal GTK-effects. Cytokine-induced cytotoxicity will then be studied as already described (Annerén & Welsh, 2001). Thus, ablation of GTK protein expression or activity prevents cytokine-induced cytotoxicity in β-cells according to the invention. This experiment can be further confirmed by assessing cytokine-induced β-cell toxicity in islets isolated from GTK knock-out mice. Potential usage of GTK-inhibition by transfection in vitro can currently be envisaged by pretreatment of islets in vitro with the GTK-inhibiting constructs above prior to transplantation (to cure type 1 diabetes). This improves the survival of transplanted β-cells and is thus beneficial in a clinical context.

### Inhibition of GTK by RNAi:

This method provides a simple measure to specifically inhibit the expression of a gene product. Basically, a double-stranded RNA oligonucleotide is introduced into cells, whereby a sequence-specific double-stranded RNase for the target mRNA is activated, thus depleting the cell of the gene product mRNA and protein. For mouse GTK, the double-stranded RNA will be the RNA sequence corresponding to AAGCGACTGGGATCTGGTCAGTT. A corresponding sequence will be chosen for the human FRK gene. This will then be transfected into the cells of interest, ie islets of Langerhans using Oligofectamin^{™}. Subsequent to transfection, GTK protein expression and sensitivity to cytokines will be measured as described below.

This procedure will be followed by in vivo measures in which the above given sequence (followed by its inversion to generate a RNA duplex) is introduced into a gene transfer virus under the control of the insulin promoter. This can then be utilized for gene therapy in order to block GTK expression specifically in beta-cells.

Inhibition of GTK-gene expression by drug treatment: It is currently not known how GTK-gene expression is regulated. By drugs that inhibit GTK-gene expression in pancreatic β-cells, a method for attenuating the cytokine-induced destruction of islet cells is obtained.
The inhibition of gene expression may be estimated by measuring the mRNA level by PCR or Northern blot.

**2. Pharmacological agents and biological agents that inhibit GTK(FRK, RAK, IYK, BSK) -kinase activity.** The specific inhibition of certain tyrosine kinases by pharmacological agents has been well described (Hanke 1996). Particularly the pyrazolopyrimidines PP1 and PP2 are both potent inhibitors of the kinase activities of certain Src-family members, with specific profiles of inhibition among the different kinases. An additional inhibitor of Src family kinases is SU6656 (Blake et al, 2000). Since GTK is also a Src-family member with certain specific characteristics, it is possible to find a pharmacologic agent with a specific inhibitory effect on GTK. Currently known kinase inhibitors can be tested in in vitro kinase assays (described below) for their relative ability to inhibit GTK. Furthermore, new inhibitors can be designed based on structural resemblance with currently known tyrosine kinase inhibitors.

### Use of pharmacological inhibitors to block GTK activity:

Screening for GTK-inhibitors among commercially available tyrosine kinase inhibitors has yielded two inhibitors, AG957 and SU4984, that inhibited GTK activity in in vitro kinase assays with a reasonably high degree of specificity. Ki using AG957 was approximately 1 µM and that of SU4984 was about 10 µM. These inhibitors were added to Islets of Langerhans in in vitro cultures together with the cytokines IL-1β + IFN-γ, and apoptosis was determined by propidium iodide staining after 24 h. It was shown that AG957 and SU 4984 prevented cytokine-induced apoptosis by 60 and 75 %, respectively, which further lends support to the view that GTK-inhibition prevents beta-cell destruction in type 1 diabetes. Other tyrosine kinase inhibitors that inhibit GTK with a fair degree of specificity are: AG 30, PD 65495 and PD 68129. It is expected that also human FRK will be inhibited.

Chemical libraries will be screened further to obtain more specific GTK inhibitors, that can be used for in vivo therapy.

*Assay for the assessment of GTK (FRK) kinase activity:* The basis for screening for specific GTK kinase inhibitors is in vitro kinase assays, utilizing baculovirus produced GTK which is purified by immunoprecipitation using the GTK antibody. Both these reagents are available for the skilled person within this field. Phosphorylation of a synthetic peptide substrate will be studied (Annerén and Welsh, 2000) testing different concentrations of inhibitors, and IC₅₀ values can then be determined for inhibition of GTK kinase activity. The specificity of drug-induced GTK-inhibition is then assessed by comparing the IC₅₀ values with those of other tyrosine kinases.

GTK-inhibition by the administration of GTK-antibodies: Antibodies that inhibit GTK kinase activity are obtained by immunizing rabbits with different domains of the GTK protein, either produced as fusion protein or as synthetic peptides. As a consequence, GTK-specific reagents are obtained. Such GTK-specific antibodies can then be delivered by liposomal techniqes to β-cells and thus may exert their action in preventing cytokine-induced β-cell destruction.

**3. Decreased GTK protein contents by selective proteolysis:** The possibility exists that certain proteolytic processes specifically regulate the cellular content of the GTK protein. An example of this is found by IKK, an inhibitor transcription factor which is rapidly degraded by selective stimulation. If such a mechanism is operating for regulation of GTK protein contents, the potential for regulating GTK activity via such mechanisms exists.

**4. Controlling of GTK signaling mechanisms:** At present, it is not known, how GTK kinase activity is regulated. If the activation mechanism for GTK normally operating in β-cells is elucidated, control of GTK-kinase activity may be excerted. The kinase activity may be influenced by extracellular ligands, and intracellular effectors in addition to second messengers (Ga²⁺, IP₃, cAMP etc). Such signaling mechanisms have the potential of control by pharmacological treatment.

### References:

Annerén, C. and Welsh, M. (2000) Role of the Bsk/Iyk non-receptor tyrosine kinase for the control of growth and hormone production in RINm5F cells. Growth Factors, 17, 233-247.
Annerén, C., Reedquist, K. A., Bos, J. L., and Welsh, M. (2000) GTK, a Src-related tyrosine kinase, induces NGF-independent neurite outgrowth in PC12 cells through activation of the Rap1 pathway. Relationship to Shb tyrosine phosphorylation and elevated levels of focal adhesion kinase. J. Biol. Chem. 275, 29153-29161.
Annerén, C. And Welsh, M. Increased cytokine-induced cytotoxicity of pancreatic islet cells from transgeneic mice expressing the Src-like tyrosine kinase GTK. Mol. Med. 7: 301-310, 2001.
Blake, R. A. et al (2000) SU6656, a selective src family kinase inhibitor, used to probe growth factor signaling. Mol. Cell. Biol. 20, 9018-9027
Hanahan, D. (1985) Heritable formation of of pancreatic beta-cell tumors in transgenic mice expressing recombinant insulin/simian virus 40 oncogenes. Nature 315, 115-122
Hanke, J. H. et al (1996) Discovery of a novel, potent, and Src family-selective tyrosine kinase inhibitor. J. Biol. Chem. 271, 695-701
Liu, D., D., et al, (2000) Cytokines induce apoptosis in beta-cells isolated from mice lacking the inducible isoform of nitric oxide synthase (iNOS-/-). Diabetes 49, 1116-1122
Mandrup-Poulsen, T. (1996) The role of interleukin-1 in the pathogenesis of IDDM. Diabetologia. 39, 1005-1029.
Welsh, M. et al (1999) Transgenic mice expressing the Shb adaptor protein under the control of rat insulin promoter exhibit altered viability of pancreatic islet cells. Mol. Med. 5, 169-180
Welsh, M. et al (2000) Role of tyrosine kinases for beta-cell replication and survival. Upsala J. Med. Sci. 105, 7-15
Öberg-Welsh, C., and M. Welsh (1995) Cloning of BSK, A murine FRK homologue with a specific pattern of tissue distribution. Gene. 152:239-242.
Öberg-Welsh, C., C. Annerén, and M. Welsh (1998) Mutation of C-terminal tyrosine residues Y497/Y504 of the Src-family member Bsk/Iyk decreases NIH3T3 cell proliferation. Growth Factors. 16:111-124.

## Claims

1. Use of the tyrosine kinase inhibitor SU 4984 3[4-(1-Formylpiperazin-4-yl)-benzylidenyl]- 2-indolinone for production of a drug for prevention/treatment of type I diabetes mellitus, wherein the inhibitor is against a FRK tyrosine kinase in pancreatic β-cells.

2. Use of FRK or the gene encoding the same, in an assay to screen for compounds for treatment of diabetes, which interfere with FRK activity in pancreatic β-cells,

3. Use of FRK or a gene encoding FRK, in a biological or chemical assay for identifying compounds for treatment of diabetes, which interfere with FRK activity in pancreatic β-cells.

## Patentansprüche

1. Verwendung des Tyrosinkinaseinhibitors SU 4984, 3-[4-(1-Formylpiperazin-4-yl)benzylidenyl]-2-indolinon, zur Herstellung eines Wirkstoffs zur Prävention/Behandlung von Diabetes mellitus Typ I, wobei der Inhibitor gegen eine FRK-Tyrosinkinase in Pankreas-β-Zellen ist.

2. Verwendung von FRK oder des Gens, welches dieselbe codiert, in einem Assay zum Screening auf Verbindungen zur Behandlung von Diabetes, welche mit der FRK-Aktivität in Pankreas-β-Zellen interferieren.

3. Verwendung von FRK oder einem Gen, das FRK codiert, in einem biologischen oder chemischen Assay zum Identifizieren von Verbindungen zur Behandlung von Diabetes, welche mit der FRK-Aktivität in Pankreas-β-Zellen interferieren.

## Revendications

1. Utilisation de l'inhibiteur de tyrosine kinase SU 4984 3[4-1(-formylpipérazin-4-yl)-benzylidényl]-2-indolinone pour la production d'un médicament pour la prévention/le traitement du diabète sucré de type I, dans laquelle l'inhibiteur est contre une tyrosine kinase FRK dans les cellules pancréatiques β.

2. Utilisation de FRK ou du gène la codant, dans un test pour cribler des composés pour le traitement du diabète, qui interfèrent avec l'activité de FRK dans les cellules pancréatiques β.

3. Utilisation de FRK ou d'un gène codant FRK, dans un test biologique ou chimique pour identifier des composés pour le traitement du diabète, qui interfèrent avec l'activité de FRK dans les cellules pancréatiques β.
